# EUROPEAN PATENT APPLICATION

(11) **EP 0 731 172 A2**
(43) Date of publication of application: **11.09.1996**
(21) Application number: 96102906.3
(22) Date of filing: 27.02.1996
(51) Int. Cl.: C12P 19/12, C12P 19/14, A23L 1/09

(54) **Process for the production of galactosyltrehalose and foods containing thereof**

(30) Priority: 09.03.1995 JP 78313/95
(71) Applicant: KUREHA CHEMICAL INDUSTRY CO., LTD., Chuo-ku Tokyo (JP)
(72) Inventor: Kase, Toshiya, Warabi-shi, Saitama (JP); Motojima, Kyoko, Kawaguchi-shi, Saitama (JP); Sakai, Jun, Kokubunji-shi, Tokyo (JP); Takahashi, Eisaku, Tokyo (JP); Konai, Yutaka, Machida-shi, Tokyo (JP)
(74) Representative: Boeters, Hans Dietrich, Dr.

(57) **Abstract**

This invention provides a process for the production of galactosyltrehalose and foods containing the same. More in detail, galactosyltrehalose is prepared by addition of β-galactosidase to lactose or a raw material containing lactose and trehalose or a raw material containing trehalose. Furthermore, foods are prepared by addition of the resultant galactosyltrehalose to raw materials of food.

## Description

### Field of the Invention

This invention relates to a process for the production of galactosyltrehalose and foods containing thereof. Galactosyltrehalose is expected to exhibits physiological activities such as anticaries activity, proliferation of bifidobacteria, stimulation of calcium absorption, immunopotentiation, and inhibition of carcinogenicity.

### Background of the Invention

Heretofore, oligosaccharides have been known to exhibit physiological activities such as anticaries activity, proliferation of bifidobacteria, stimulation of calcium absorption, immunopotentiation, and inhibition of carcinogenicity. Galactosyltrehalose, one of galacto-oligosaccharides, is a non-reducing oligosaccharide composed of galactose and two molecules of glucose. Galactosyltrehalose is known to be formed from galactose and trehalose by the action of β-galactosidase [Carbohydrate Research, 199, 227-234 (1990)]. However, the method mentioned above has a drawback of low yield of 5.1 mole % from the raw material trehalose and no effective process for the production of galactosyltrehalose has been known. Furthermore, no food or drink containing galactosyltrehalose has been known.

The inventors of the present invention in view of these conditions have been investigating on an easily operable process for the production of galactosyltrehalose in high yield, found galactosyltrehalose can be prepared from lactose and trehalose by the action of β-galactosidase, and accomplished the present invention.

### Summary of the Invention

Therefore, an object of the present invention is to provide a process for the production of galactosyltrehalose from lactose or a raw material containing lactose and trehalose or a raw material containing trehalose by the addition of β-galactosidase.

Another object of the present invention is to provide foods containing galactosyltrehalose prepared from lactose or a raw material containing lactose and trehalose or a raw material containing trehalose by adding β-galactosidase.

### Brief Description of Drawings

Fig. 1 shows the profile of HPLC (high performance liquid chromatography) of sugar solution after the reaction according to Example 1.

Peaks found at elution times of 10.4, 15.9 and 29.3 minute show glucose, lactose and trehalose, and galactosyl-trehalose, respectively.

Fig. 2 shows the ¹³C-NMR spectrum of galactosyl-trehalose according to Example 2.

### Detailed Description of the Invention

### Preferred Embodiment

In the present invention, lactose or materials containing lactose may include lactose-containing products such as milk, commercially available lactose, skim milk and whey. Trehalose or materials containing trehalose may be illustrated with commercial available trehalose, yeast containing trehalose, fermented mixture containing trehalose, trehalose-forming enzymic reaction mixtures or the like. β-galactosidase derived from any origin can be used in the present invention. Exemplified are β-galactosidases derived from Aspergillus, yeast, lactic acid bacteria and the like. Additionally, crude enzyme may be used as well as purified enzyme. Furthermore the enzyme may be applied in a form of immobilized enzyme.

In the present invention, the concentrations of lactose or raw materials containing lactose, and trehalose or raw materials containing trehalose are not limitative and any concentration which substantially forms galactosyltrehalose may be selected. Lactose or materials containing lactose are used as solutions at concentrations of 1-50 weight % and trehalose or materials containing trehalose are used as solutions at concentrations of 1-50 weight %. The combination ratios of trehalose to one weight part of lactose are 0.2-5, preferably 0.5-2.0. The amount of β-galactosidase used for the reaction is not restrictive and 1-1,000 unit/ml, preferably 10-600 unit/ml is used. The enzymic reaction is carried out at pH 3-9 and at 10-80°C, preferably at 20-50°C for 0.5 hr. to several days.

In the above mentioned enzyme reaction, galactose residue in lactose transfers to trehalose to form galactosyltrehalose. Galactosyltrehalose formed by transgalactosylation of the present invention is mainly composed of O-β-D-galactopyranosyl-(1,6)-O-α-D-glucopyranosyl-(1,1)-α-D-glucose. In addition, a galactosyltrehalose in which galactose residue binds to C-4 or C-3 of trehalose is also formed.

The reaction mixture containing the formed galactosyltrehalose as shown above is thermally treated to inactivate the enzyme and then a solution containing galactosyltrehalose is obtained.

The transglycosylation of galactose residue from lactose proceeds even in the presence of ethanol, and galactosyltrehalose can be formed in alcoholic beverages. The hydrolytic reaction of lactose in the presence of ethanol proceeds comparatively slower than that in an aqueous solution and formation of galactosyltrehalose by the transglycosylation can be effectively carried out. In those reactions, the concentration of ethanol in the enzymic reaction system is held at 30% or lower.

The solution containing galactosyltrehalose obtained by the transglycosylation can be used as foods and drink as it is. Alternatively, galactosyltreharose can be separated from the solution using conventional method. The galactosyltreharose separated or the galactosyltreharose-containing solution may be added to the other foods and drinks to prepare foods and drinks containing galactosyltrehalose, for example, confectionery such as biscuits, candies, chewing gums, chocolates and snacks, breads, noodles including buckwheats, Chinese noodles, rice noodles, fine noodles and convenience noodles, canned vegetables such as bamboo shoots, asparagus, mushrooms and edible fruit bodies of fungi, canned fruits such as mandarins, peaches, apples, figs and pears, processed horticultural products such as marmalades, jams, dried fruits and vegetables, bean curds or processed bean curds, agricultural products such as soy bean protein, margarines, fermented foods such as liquors and soy sauce, fermented pasty and sticky products of soy beans (miso and natto), pickles, milk products such as condensed and powder milks, butters, cheeses and ice creams, lactobacillus products such as yogurt, livestock products such as hams, sausages and canned meats, processed fish meat products such as steamed or fried fish meat and fish meat sausages, and fishery foods such as dried fish, fish meat, fish eggs, shrimps and sea shells.

As described above, galactosyltrehalose in which galactose residue of lactose is transferred to trehalose can be prepared according to the present invention in high yield. The resultant galactosyltrehalose is expected to stimulate the growth of bifidobacteria, lower the content of cholesterol and neutral fat, and prevent dental caries as well as known galactosyltrehalose, and can be used for the production of various foods and drinks with added galactosyltrehalose. In addition, galactosyltrehalose is expected to improve stability and shelf life of enzymes and proteins, and stabilize foods and drinks during drying and freezing procedures.

The present invention will be practically explained by the examples, however, the scope of the present invention is not restricted by these examples.

### [Example 1]

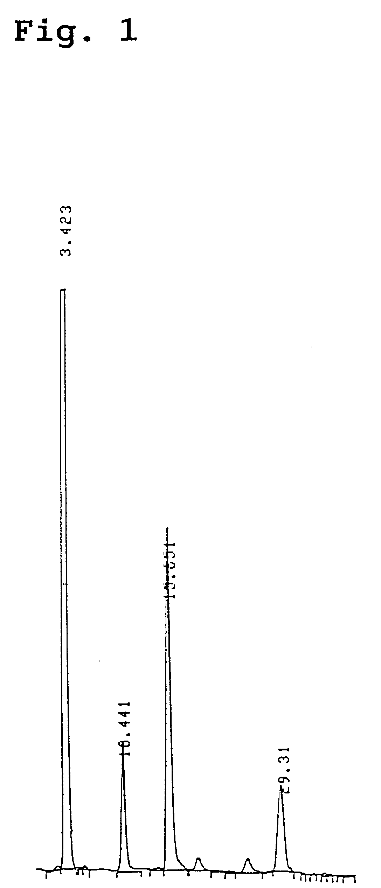

A reaction mixture (10 ml) composed of 50 mM phosphate buffer (pH 7.0), 2.4 g of lactose, 2.5 g of trehalose and 4,000 unit of Escherichia coli derived β-galactosidase was incubated at 35°C for 14 hrs. Then, the reaction mixture was analyzed by HPLC and the profile thereof is shown in Fig. 1. The concentration of galactosyltrehalose formed as a main component was about 80 mg/ml with a yield of 21.8 mole % to the raw material trehalose.

### [Example 2]

The whole reaction mixture containing galactosyl-trehalose formed by Example 1 was charged to a column packed with active charcoal to adsorb disaccharides or higher oligosaccharides. The saccharide-adsorbed column was washed with purified water and eluted with 50% ethanol. The eluate was condensed and fractionated with HPLC. The fractionation was carried out using a polyamine column (YMC pack™ Polyamine II 10 mm x 250 mm, YMC Co., Ltd.), eluting solution of acetonitrile:water = 65:35, and flow rate of 5 ml/min.
Fractions at retention time of 13.5-15 minutes were collected and evaporated to give 710 mg of white powder with a yield of 19.3 mole % to the raw material trehalose.

Spectra of ¹H-NMR and ¹³C-NMR of the white powder obtained above clearly indicated the β-bonding of 1st position carbon of galactose residue (shift value: 106.15 ppm) with 6th position carbon of trehalose (shift value: 70.97 ppm) from the coupling constant of 1st position hydrogen of galactose (J = 3.5 Hz).

Hydrolysis of the white powder with 0.05N sulfuric acid gave one mole of galactose and two moles of glucose. Furthermore, partial hydrolysis gave three carbohydrates of galactose, glucose and trehalose. The white powder was not reduced by Somogyi-Nelson method test.

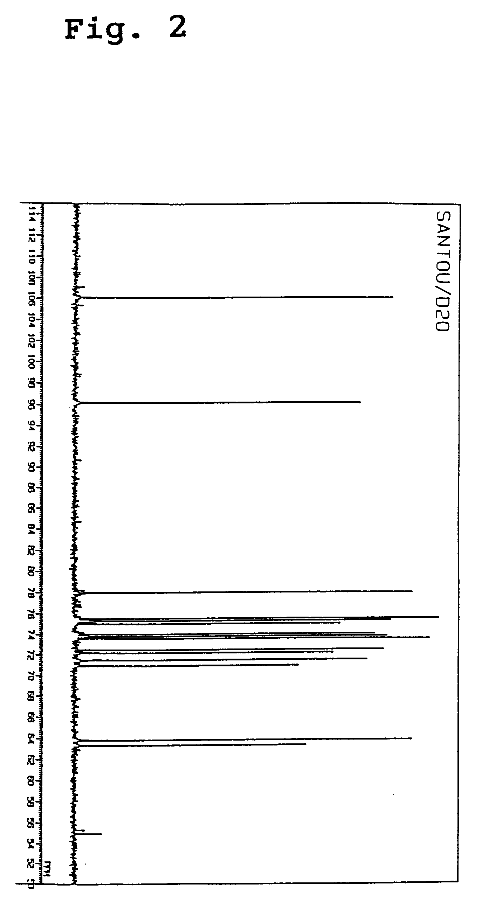

These results showed that the white powder has a chemical structure of O-β-D-galactopyranosyl-(1,6)-O-α-D-glucopyranosyl-(1,1)-α-D-
glucose. The ¹³C-NMR spectrum of the white powder is shown in Fig. 2.

### [Example 3]

A mixture of 40 µl of 1M MES buffer (pH 6.0), 250 µl of 0.5M each of lactose and trehalose solution, 10 µl of 1.9 unit/ml of Escherichia coli derived β-galactosidase solution, 25 µl of ethanol and 75 µl of purified water was incubated at 30°C for 23 hrs. After the reaction, the reaction mixture of sugar solution was analyzed with HPLC.
The concentration of galactosyl-trehalose in the reaction mixture was 34 mM.

### [Example 4]

In 200 ml of water, 48 g of lactose and 50 g of trehalose were dissolved and adjusted to pH 7.0, then 8,000 units of β-galactosidase was added and incubated at 35°C for 24 hrs. After the reaction, the enzyme was inactivated and 20 g of galactosyltrehalose fraction was obtained by active charcoal chromatography and preparative HPLC.

In 1,000 ml of fresh milk with added 15 g of skim milk powder, 10 g of galactosyltrehalose prepared above was added and pasteurized at 98°C for 3 minutes. The resultant formulated milk was inoculated with 4 ml of a starter composed of 10⁸ cells/ml of Lactobacillus bulgaricus and 10⁹ cells/ml of Streptococcus thermophilus and incubated at 40°C for 9 hrs. The resultant yogurt showed smooth texture, mild flavor and pH 4.7, indicating production of favorable yogurt food containing galactosyltrehalose.

### [Example 5]

A mixture of 53 weight parts of wheat flour, three weight parts of galactosyltrehalose, eight weight parts of a shortening and 26 weight parts of sugar was ground. Then, one weight part each of egg and a baking powder, and eight weight parts of water were added, kneaded and molded. The molded doughs were baked at 180°C for 10-15 minutes to give biscuits containing galactosyltrehalose

### [Example 6]

Twenty weight parts of salt free butter was melted to give cream and 18 weight parts of sugar, 19 weight parts of egg and a small amount of vanilla essence were mixed thoroughly. Furthermore, 40 weight parts of soft flour, one weight part of a baking powder and two weight parts of galactosyltrehalose were added, kneaded to give doughs. The doughs were molded and baked for 10-15 minutes in an oven preheated at 170-180°C to give cookies containing galactosyltrehalose.

### [Example 7]

A mixture of 20 weight parts of vinyl acetate resin with average polymerization degree of 650, 30 weight parts of polyisobutylene with average molecular weight of 300,000, five weight parts of an ester gum and 20 weight parts of wax was kneaded at 110-120°C till the gum base melted. The melted mixture was kneaded further for about one hr. Then seven weight parts of fatty acid monoglyceride, six weight parts of acetylated monoglyceride and 12 weight parts of calcium carbonate were added, kneaded for 15 minutes and cooled to ordinary temperature to give a gum base. A mixture of 25 weight parts of the gum base, 51 weight parts of powdered sugar, five weight parts of glucose, 12 weight parts of corn syrup, one weight part each of glycerin and a flavor, and five weight parts of galactosyltrehalose was kneaded for 15 minutes, rolled and cut to give chewing gums containing galactosyltrehalose.

### [Example 8]

In 14.5 weight parts of milk, one weight part of galactosyltrehalose, 0.1 weight part of an yeast food, one weight part of salt, six weight parts each of sugar and egg were thoroughly mixed. The resultant mixture was homogeneously mixed with 58 weight parts of well sieved hard flour. Furthermore, a suspension prepared from 12 weight parts of water, one weight part of dried yeast and 0.4 weight part of sugar was added, thoroughly kneaded till no farinaceous feeling was observed. Then, 20 weight parts of butter was added portionwise to give a homogenous mixture, kneaded and pounded several hundred times. The resultant dough was fermented at about 30°C for about one hr., divided, rounded to give smooth surface. The divided and smoothly rounded doughs were allowed to stand at 30°C for 15 minutes, rolled with a rolling pin to give triangular thin shape. The triangular doughs were rolled from the wider side, placed on a pan thinly spread with an edible oil so as to face the joint to the pan, baked for about 30 minutes, spread with egg yolk and baked in an oven preheated at 180°C for 15 minutes to give butter rolls containing galactosyltrehalose.

## Claims

1. A process for the production of galactosyltrehalose characterized by addition of β-galactosidase to lactose or a raw material comprising lactose and trehalose or a raw material comprising trehalose to form galactosyltrehalose.

2. Foods and drinks comprising galactosyltrehalose prepared by addition of β-galactosidase to lactose or a raw material comprising lactose and trehalose or a raw material comprising trehalose.

3. Foods and drinks comprising galactosyltrehalose characterized by preparing galactosyltrehalose by addition of β-galactosidase to lactose or a raw material containing lactose and trehalose or a raw material containing trehalose, followed by mixing said galactosyltrehalose with foods.

4. Galactosyltrehalose containing foods according to Claim 2 or 3, wherein galactosyltrehalose containing foods are agricultural foods, fermented foods, livestock foods or fishery foods.

5. The food according to Claim 2 or 3 wherein galactosyltrehalose containing food is a chewing gum.
